(19)

Europäisches
Patentamt
European
Patent Office
Office européen
des brevets

(11) **EP 3 388 140 A1**

(12) **EUROPEAN PATENT APPLICATION**

(43) Date of publication:
17.10.2018   Bulletin 2018/42

(21) Application number: 17165657.2

(22) Date of filing: 10.04.2017

(51) Int Cl.:
B01D 63/02 (2006.01)          A61M 1/36 (2006.01)
B01D 69/14 (2006.01)          B01D 71/76 (2006.01)
B01D 71/82 (2006.01)          B01D 71/42 (2006.01)
B01D 71/60 (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Gambro Lundia AB
220 10 Lund (SE)**

(72) Inventor: **Harenski, Kai
80638 München (DE)**

(74) Representative: **Perchenek, Nils
Gambro Dialysatoren GmbH
Legal and Intellectual Property
Holger Crafoord-Strasse 26
72379 Hechingen (DE)**

(54) **EXTRACORPOREAL BLOOD CIRCUIT**

(57)      The present disclosure relates to extracorporeal blood circuits used for gas exchange in blood, in particular circuits for cardiopulmonary bypass.

**EP 3 388 140 A1**

**Description**

**Technical Field**

The present disclosure relates to extracorporeal blood circuits used for gas exchange in blood, in particular circuits for cardiopulmonary bypass.

**Description of the Related Art**

Cardiopulmonary bypass (CPB) is commonly used in various surgical procedures performed on the heart or the lung of a patient. Examples include coronary artery bypass surgery, cardiac valve repair and/or replacement, repair of large septal defects, repair and/or palliation of congenital heart defects, transplantation, pulmonary thromboendarterectomy, or pulmonary thrombectomy. A machine pumps the blood, and, using an oxygenator, allows red blood cells to pick up oxygen, as well as allowing carbon dioxide levels to decrease. This mimics the function of the heart and the lungs, respectively. CPB mechanically circulates and oxygenates blood for the body while bypassing the heart and lungs. It uses a heart-lung machine (HLM) to maintain perfusion to other body organs and tissues while the surgeon works in a bloodless surgical field.

During the surgical procedure, excessive water has to be removed from the patient, e.g., by ultrafiltration. Hemo-filters are commonly used for this purpose on heart-lung machines.

Extracorporeal membrane oxygenation (ECMO), also known as extracorporeal life support (ECLS), is an extracorporeal technique of providing both cardiac and respiratory support to persons whose heart and lungs are unable to provide an adequate amount of gas exchange to sustain life. ECMO works by removing blood from the person's body and artificially removing the carbon dioxide and oxygenating red blood cells. In veno-arterial (VA) ECMO, this blood is returned to the arterial system and in veno-venous (VV) ECMO the blood is returned to the venous system.

During extracorporeal blood circulation, inflammatory mediators such as cytokines and endotoxins are produced that can lead to a systemic inflammatory response. Multiple measures have been developed over the years to reduce inflammatory release. More recently, it has been proposed to integrate an adsorptive column capturing cytokines into the extracorporeal circuit.

**Summary**

The present disclosure provides an extracorporeal circuit in which ultrafiltration and the adsorption of inflammatory mediators is combined in a single device.

**Detailed Description**

An extracorporeal blood circuit is provided which comprises a gas exchange device and a diffusion- and/or filtration device. The diffusion- and/or filtration device comprises semipermeable membranes comprising i) a copolymer of acrylonitrile and sodium methallyl sulfonate, ii) a polyethyleneimine, and iii) heparin. In one embodiment of the extracorporeal circuit of the present disclosure, the diffusion- and/or filtration device is a capillary dialyzer comprising a plurality of semipermeable hollow fiber membranes, the membranes comprising i) a copolymer of acrylonitrile and sodium methallyl sulfonate; ii) a polyethyleneimine; and iii) heparin.

Diffusion- and/or filtration devices comprising semipermeable membranes comprising i) a copolymer of acrylonitrile and sodium methallyl sulfonate, ii) a polyethyleneimine, and iii) heparin are known in the art.

US 2003/0021826 A1 discloses binding an anticoagulation agent in a stable manner to the surface of semipermeable membranes essentially comprised of a copolymer of acrylonitrile and at least one anionic and anionizable monomer. A semi-permeable composite membrane is disclosed which comprises a semi-permeable support membrane and an anticoagulation agent suitable for the treatment of blood or plasma by extracorporeal circulation, said semipermeable support membrane being essentially constituted by a polyacrylonitrile carrying anionic or anionizable groups; the surface of the semipermeable support membrane intended to be brought into contact with the blood or plasma is coated in succession with a cationic polymer carrying cationic groups which can form an ionic bond with anionic or anionizable groups of polyacrylonitrile, the cationic polymer (for example polyethyleneimine, PEI) comprising chains of a size which is sufficient not to traverse the semi-permeable support membrane, and an anticoagulation agent carrying anionic groups which are capable of forming an ionic bond with cationic groups of said cationic polymer (for example heparin).

WO 2007/148147 A1 describes the use, on a membrane preferably based on a copolymer of acrylonitrile and sodium methallyl sulfonate, of a solution of a polymer carrying anionic or anionizable groups in the colloidal form and in an acidic medium, in particular by mixing, for example, a solution of polymer carrying anionic or anionizable groups with a solution of organic polyacid in a specific proportion with respect to said polymer, which results in an increase in both the quantity of polymer grafted to the surface of the membrane and the availability of free cationic or cationizable groups at the surface of this membrane coating. The membrane described allows a large quantity of compounds carrying anionic or anionizable groups to be bound. It is suggested for the treatment of septic syndrome, in particular for adsorbing endotoxins contained in the biological fluid, for purifying certain molecules contained in the blood or the plasma by extracorporeal circulation and for reducing systemic anticoagulation in a patient during an extracorporeal blood or plasma treatment. A method for preparing the membrane is also described in WO 2007/148147 A1.

The diffusion- and/or filtration device removes excessive water from the patient by ultrafiltration and also adsorbs inflammatory mediators from the blood of the patient. Examples of suitable diffusion- and/or filtration devices include commercial capillary dialyzers available from Gambro Lundia AB under the trade names oXiris® and Evodial®.

One example for a current product comprising a AN69 type membrane is the Evodial dialyzer, which is a hemodialyzer equipped with a heparin-grafted acrylonitrile based membrane such as described in the aforementioned WO 2007/148147 A1 (the so-called HeprAN membrane). The Evodial membrane is characterized also in that the charged surface, originating from anionic sulfonate groups, is neutralized by the polycationic biopolymer polyethyleneimine. The surface treatment also allows the almost irreversible fixing of said heparin through very strong ionic binding between the negative charges of heparin and the free positive charges of the cationic polymer. Membranes having the ability to immobilize heparin are highly desirable as it further reduces the need of systemic doses of heparin.

In one embodiment of the extracorporeal blood circuit, the membranes of the diffusion- and/or filtration device comprise polyethyleneimine in an amount of from 10 to 100 mg, for instance, from 25 to 50 mg per $m^2$ of membrane surface area, and from 1,500 to 10,000 UI, for instance, 3,000 to 6,000 UI, e.g. 4,500±1,500 UI, of heparin per $m^2$ of membrane surface area.

In one embodiment, the diffusion- and/or filtration device comprises hollow fiber membranes having an inner diameter in the range of from 180 to 260 $\mu$m, e.g. 210 $\mu$m, or 240 $\mu$m. In one embodiment, the wall strength of the hollow fiber membranes is in the range of from 30 to 60 $\mu$m, e.g., 40 to 50 $\mu$m. In one embodiment, the overall surface area of the membranes in the diffusion- and/or filtration device is in the range of from 1 to 3 $m^2$, for instance, 1.0 to 2.2 $m^2$.

In one embodiment, the hollow fiber membranes in the diffusion- and/or filtration device show sieving coefficients, measured at 37°C in bovine plasma having a protein content of 60 g/l, of > 0.95 for inulin; > 0.55 for myoglobin; and < 0.01 for albumin.

The membranes in the diffusion- and/or filtration device have a remarkable ability to immobilize inflammatory mediators and endotoxins to their surface by adsorption. In one embodiment, the clearance rate for IL-6 in HDF mode with $Q_B$=400ml/min, $Q_D$=700ml/min and UF= 100 ml/min is in the range of from 20 to 40 ml/min.

The extracorporeal blood circuit of the present disclosure also comprises a gas exchange device. The gas exchange device performs gas exchange in the blood of the patient, i.e., it removes carbon dioxide from the blood and/or oxygenates the blood. In one embodiment of the extracorporeal circuit, the gas exchange device is a membrane oxygenator. Membrane oxygenators are known in the art, and a large variety of devices is commercially available. The membrane oxygenator comprises semipermeable polymer membranes. Examples of suitable polymers include polypropylene, polyethylene, poly-4-methylpentene (PMP), polyvinylidene fluoride (PVDF), and polytetrafluoroethylene (PTFE). In another embodiment, the gas exchange device is a bubble oxygenator. Bubble oxygenators also are known in the art, and commercially available.

In one embodiment, the extracorporeal blood circuit of the present disclosure is a cardiopulmonary bypass (CPB). In one embodiment, the cardiopulmonary bypass is a veno-arterial bypass. In another embodiment, the cardiopulmonary bypass is a veno-venous bypass. In one embodiment, the extracorporeal blood circuit comprises a heart-lung machine (HLM). In another embodiment, the extracorporeal blood circuit is configured for extracorporeal membrane oxygenation (ECMO). In one embodiment, the extracorporeal blood circuit is configured for veno-arterial (VA) ECMO. In another embodiment, the extracorporeal blood circuit is configured for veno-venous (VV) ECMO.

The present disclosure also concerns the use of a diffusion- and/or filtration device comprising semipermeable membranes in cardiopulmonary bypass or ECMO. The membranes comprise i) a copolymer of acrylonitrile and sodium methallyl sulfonate; ii) a polyethyleneimine; and iii) heparin. In one embodiment, the cardiopulmonary bypass comprises a membrane oxygenator. In a further embodiment, the cardiopulmonary bypass comprises a heart-lung machine.

When blood from a patient passes through the extracorporeal circuit of the present disclosure, the gas exchange device removes carbon dioxide from the blood and/or oxygenates it, i.e., replenishes it with oxygen. Inflammatory mediators and endotoxins are adsorbed by the diffusion- and/or filtration device; and water is withdrawn from the bloodstream by ultrafiltration. It is an advantage of the extracorporeal circuit of the present disclosure that removal of inflammatory mediators and endotoxins as well as removal of excess water from the patient are accomplished using a single diffusion- and/or filtration device. This greatly simplifies the assembly and operation of the extracorporeal circuit. It is hypothesized that in comparison to a cardiopulmonary bypass which only features a hemofilter for removing excess water, the additional removal of inflammatory mediators and endotoxins positively affects the outcome of surgical procedures requiring CPB.

It will be readily apparent to one skilled in the art that various substitutions and modifications may be made to the invention disclosed herein without departing from the scope and spirit of the invention.

The present invention is illustrated by way of non-limiting examples which are further detailed in the Figures accompanying them in order to further facilitate the understanding of the invention.

**Examples**

Removal of inflammatory mediators and endotoxins by a capillary dialyzer having an effective surface area of 1.5 m$^2$ (oXiris®, Gambro Lundia AB) was tested. The dialyzer comprises hollow fiber membranes comprising a copolymer of acrylonitrile and sodium methallyl sulfonate. The surface of the fibers comprises 30 mg/m$^2$ of high MW polyethylene-imine (PEI) and is grafted with 4,500 ± 1,500 UI/m$^2$ of heparin. The fibers have an inner diameter of 240 $\mu$m and a wall strength of 40 $\mu$m.

Interleukin-10 (IL-10; MW=18 kDa), Interleukin 6 (IL-6; MW=26 kDa), High-mobility group box 1 (HMGB-1; MW=30 kDa) and Tumor necrosis factor alpha (TNF-a; MW=51 kDa) were used as cytokines. Lipopolysaccharide (LPS; MW>100 kDa) was used as endotoxin.

The *in vitro* experiments were conducted using a pool of 500 ml of frozen human plasma with a protein content of 60±5 g/l from healthy volunteers stabilized with a LPS theoretical concentration of [25 - 75] EU/mL. Spiking of IL-10, IL-6, HMGB-1 and TNF-a cytokines at a respective concentration of 500 pg/mL, 1,500 pg/mL, 30 ng/mL and 250 pg/mL was performed prior to the start of circulation.

A number of n=3 samples was selected (including n=3 multiple batches), which has been found adequate to demonstrate a systematic removal mechanism and considering that clinical outcomes related to endotoxin/cytokine removal may significantly differ depending on the patient pathological conditions (no direct clinical correlation between the foreseen cytokine adsorbed quantity and the plasma final circulating cytokine level). The circulation conditions are representative of standard CRRT practices.

Prior to circulation of the plasma pool, the blood compartment of the dialyzer was washed with 1.5 L of heparinized (5 UI/mL) saline solution at 150 ml/min. Additional 500 ml of the solution were used to rinse the dialysate compartment by ultrafiltration.

The plasma pool was circulated in a closed loop for 120 min at 150 ml/min. Sampling was performed during the circulation at baseline and after t=5, t=10, t=30, t=60 and t=120 min directly from the plasma pool.

Concentration of cytokines was measured in duplicate by enzyme-linked immunosorbent assay (ELISA) utilizing matched antibody pairs and recombinant (Quantikine R&D system, France; and IBL international, Germany for human HMGB-1). LPS concentration was measured in duplicate by limulus amoebocyte lysate (LAL) chromogenic method (K-QCL Lonza assay).

Removal Rate specifications at the end of the circulation (t=120 min) are calculated for each mediator using:

$$RR_{(t=120\ min)} = [1 - C_{(t=120\ min)} / C_{(t=0\ min)}] \times 100$$

with

$C_{(t=0\ min)}$ :      initial concentration in the plasma pool (baseline sample)
$C_{(t=120\ min)}$ :      final concentration in the plasma pool at t=120 min

The results are summarized in Table 1.

Tab.1 Mean calculated removal rate at t=120 min for IL-10, IL-6, HMGB-1, TNF-a cytokines and LPS

| | Cytokine adsorption RR (%) | | | | LPS adsorption RR (%) |
|---|---|---|---|---|---|
| | IL-10 | IL-6 | HMGB-1 | TNF-$\alpha$ | |
| Batch 1 | 94,1 | 82,9 | 93,0 | 84,5 | 73,6 |
| Batch 2 | 97,9 | 82,0 | 92,7 | 77,4 | 77,8 |
| Batch 3 | 97,0 | 82,8 | 99,3 | 60,2 | 82,0 |
| **Mean (n=3)** | **96,3** | **82,6** | **95,0** | **74,0** | **77,8** |
| SD | 2,0 | 0,5 | 3,7 | 12,5 | 4,2 |

(continued)

| | Cytokine adsorption RR (%) | | | | LPS adsorption RR (%) |
|---|---|---|---|---|---|
| | IL-10 | IL-6 | HMGB-1 | TNF-$\alpha$ | |
| CV (%) | 2,1 | 0,6 | 3,9 | 16,9 | 5,4 |
| *calculated at t=120 min | | | | | |

The *in vitro* experiments confirm the high affinity of the oXiris® membrane to the different cytokines challenged (IL-10 / IL-6 / HMGB-1 and TNF-a) as well as to the endotoxin LPS. Those observations appear consistent with available clinical evidence, suggesting the capability of the device for selective elimination of inflammatory mediators and associated improved patient condition (improved SOFA score and cardiovascular function).

**Claims**

1. An extracorporeal blood circuit comprising

   a) a gas exchange device; and
   b) a diffusion- and/or filtration device comprising semipermeable membranes, the membranes comprising i) a copolymer of acrylonitrile and sodium methallyl sulfonate, ii) a polyethyleneimine, and iii) heparin.

2. The extracorporeal blood circuit of claim 1, wherein the gas exchange device is a membrane oxygenator.

3. The extracorporeal blood circuit of claim 1 or 2, wherein the extracorporeal blood circuit is a cardiopulmonary bypass (CPB).

4. The extracorporeal blood circuit of claim 3, wherein the blood circuit comprises a heart-lung machine (HLM).

5. The extracorporeal blood circuit of any one of claims 1 to 3, wherein the extracorporeal blood circuit is configured for extracorporeal membrane oxygenation (ECMO).

6. The extracorporeal blood circuit of claim 3, wherein the cardiopulmonary bypass is a veno-arterial bypass.

7. The extracorporeal blood circuit of claim 3, wherein the cardiopulmonary bypass is a veno-venous bypass.

8. The extracorporeal blood circuit of any one of the preceding claims, wherein the diffusion- and/or filtration device is a capillary dialyzer comprising a plurality of semipermeable hollow fiber membranes, the membranes comprising i) a copolymer of acrylonitrile and sodium methallyl sulfonate; ii) a polyethyleneimine; and iii) heparin.

9. Use of a diffusion- and/or filtration device comprising semipermeable membranes, the membranes comprising i) a copolymer of acrylonitrile and sodium methallyl sulfonate; ii) a polyethyleneimine; and iii) heparin, in a cardiopulmonary bypass.

10. The use of claim 8, wherein the cardiopulmonary bypass comprises a membrane oxygenator.

11. The use of claim 8 or 9, wherein the cardiopulmonary bypass comprises a heart-lung machine.

Europäisches
Patentamt

European
Patent Office

Office européen
des brevets

# EUROPEAN SEARCH REPORT

Application Number

EP 17 16 5657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 99/47190 A1 (MEDTRONIC INC [US]; ERICSON DANIEL G [US]; THOR ERIC J [US]; HAWORTH W) 23 September 1999 (1999-09-23)<br>* abstract *<br>* figure 2 *<br>* page 20, lines 5-10 *<br>* page 9, lines 5-7 *<br>* page 10, line 4 - page 11, line 16 *<br>* page 16, lines 1-15 *<br>----- | 1-11 | INV.<br>B01D63/02<br>A61M1/36<br>B01D69/14<br>B01D71/76<br>B01D71/82<br>B01D71/42<br>B01D71/60 |
| Y,D | WO 2007/148147 A1 (GAMBRO LUNDIA AB [SE]; THOMAS MICHEL [FR])<br>27 December 2007 (2007-12-27)<br>* abstract *<br>* figures 8,9 *<br>* page 2, lines 13-18 *<br>* claims 13-34 *<br>----- | 1-11 | |
| A,D | US 2003/021826 A1 (CROST THIERRY [FR] ET AL) 30 January 2003 (2003-01-30)<br>* the whole document *<br>----- | 1-11 | |
| | | | TECHNICAL FIELDS SEARCHED (IPC)<br><br>B01D<br>A61M |

The present search report has been drawn up for all claims

| Place of search | Date of completion of the search | Examiner |
|---|---|---|
| Munich | 19 September 2017 | Lançon, Eveline |

EPO FORM 1503 03.82 (P04C01)

## ANNEX TO THE EUROPEAN SEARCH REPORT
## ON EUROPEAN PATENT APPLICATION NO.

EP 17 16 5657

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2017

| Patent document cited in search report | | | Publication date | Patent family member(s) | | | Publication date |
|---|---|---|---|---|---|---|---|
| WO | 9947190 | A1 | 23-09-1999 | AU | 3006199 | A | 11-10-1999 |
| | | | | EP | 1061972 | A1 | 27-12-2000 |
| | | | | US | 6733471 | B1 | 11-05-2004 |
| | | | | US | 2004181184 | A1 | 16-09-2004 |
| | | | | US | 2007098593 | A1 | 03-05-2007 |
| | | | | WO | 9947190 | A1 | 23-09-1999 |
| WO | 2007148147 | A1 | 27-12-2007 | AU | 2006344976 | A1 | 27-12-2007 |
| | | | | CA | 2655624 | A1 | 27-12-2007 |
| | | | | CN | 101505858 | A | 12-08-2009 |
| | | | | EP | 2040827 | A1 | 01-04-2009 |
| | | | | ES | 2532256 | T3 | 25-03-2015 |
| | | | | FR | 2902670 | A1 | 28-12-2007 |
| | | | | JP | 5199252 | B2 | 15-05-2013 |
| | | | | JP | 2009540903 | A | 26-11-2009 |
| | | | | KR | 20090029791 | A | 23-03-2009 |
| | | | | KR | 20130002352 | A | 07-01-2013 |
| | | | | US | 2009206038 | A1 | 20-08-2009 |
| | | | | WO | 2007148147 | A1 | 27-12-2007 |
| US | 2003021826 | A1 | 30-01-2003 | AT | 449640 | T | 15-12-2009 |
| | | | | AU | 779469 | B2 | 27-01-2005 |
| | | | | CA | 2368999 | A1 | 02-08-2001 |
| | | | | EP | 1165214 | A1 | 02-01-2002 |
| | | | | ES | 2337231 | T3 | 22-04-2010 |
| | | | | FR | 2804328 | A1 | 03-08-2001 |
| | | | | JP | 4234345 | B2 | 04-03-2009 |
| | | | | JP | 2003520656 | A | 08-07-2003 |
| | | | | US | 2003021826 | A1 | 30-01-2003 |
| | | | | WO | 0154802 | A1 | 02-08-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030021826 A1

- WO 2007148147 A1